# EUROPEAN PATENT SPECIFICATION

(11) **EP 3 402 526 B1**
(45) Date of publication and mention of the grant of the patent: **24.03.2021**
(21) Application number: 17701947.8
(22) Date of filing: 09.01.2017
(51) Int. Cl.: A61K 9/20, A61K 9/48, A61K 47/10, A61K 47/14, A61K 31/223, A61P 1/00, A61P 1/12

(54) **NEW IMPROVED COMPOSITION COMPRISING AT LEAST ONE CADOTRIL**
NEUE VERBESSERTE ZUSAMMENSETZUNG MIT MINDESTENS EINEM CADOTRIL
NOUVELLE COMPOSITION AMÉLIORÉE COMPRENANT AU MOINS UN CADOTRIL

(30) Priority: 13.01.2016 SE 1650033
(43) Date of publication of application: 21.11.2018
(73) Proprietor: Johnson & Johnson Consumer Inc., Skillman, NJ 08558 (US)
(72) Inventor: MUHAMMED, Salih Muhsin, 26574 Hyllinge (SE); LINDELL, Katarina, 24193 Eslov (SE); HUGERTH, Andreas, 23735 Bjarred (SE)
(74) Representative: Kirsch, Susan Edith
(86) International application number: PCT/US2017/012665
(87) International publication number: WO 2017/123485

(56) References cited:
- WO-A1-95/28921
- WO-A1-2016/069871
- US-A1- 2015 342 882
- DATABASE WPI Section Ch, Week 201153 Thomson Scientific, London, GB; Class A96, AN 2011-G21034 XP002768176, LIU X; WANG B; ZHANG Y: "Medicine composition used for curing diarrhea, comprises racecadotril, berberine hydrochloride, and auxiliary materials", -& CN 102 018 707 A (KUNMING BANGYU PHARM CO LTD) 20 April 2011 (2011-04-20)

## Description

### FIELD OF INVENTION

The invention relates to a semi-solid composition comprising at least one cadotril and at least one liquid-lipid and/or propylene glycol excipient, a dose unit comprising the semi-solid composition as well as the above semi-solid composition or the above dose unit for use in treating a subject suffering from a disease or disorder in the gastro intestinal tract.

### BACKGROUND OF INVENTION

Diarrhea is an intestinal disorder that is characterized by an increase in the frequency of watery bowel movements. It may result from a variety of causes including bacteria or viral induced diarrhea. Food intolerance caused by allergy or the consumption of foods such as fatty or spicy foods may result in diarrhea. Food poisoning may also lead to diarrhea. In some instances, diarrhea may be a symptom of other conditions and diseases. One example is the irritable bowel syndrome (IBS). A patient with IBS typically presents clinically with one of three variants: i) chronic abdominal pain and constipation (also known as spastic colitis); ii) chronic intermittent diarrhea, often without pain; or iii) both features, in an alternating cycle of constipation and diarrhea.

Diarrhea is symptomatic of an intestinal or other bodily function disorder. Various prescription and nonprescription products can be taken for relief. However, many of these products provide relief with some side effects.

Racecadotril is a cadotril compound that is used in the treatment of diarrhea. It reduces (i) hypersecretion of water and electrolytes into the intestinal lumen, (ii) the incidence and duration of acute diarrhea and (iii) diarrhea-associated symptoms.

US2015/0342882 discloses the use of liquid formulations comprising cadotril compounds, but is silent about semi- solid formulations.

Tran & Wang, 2014, Front Chem Sci.Eng 8(2):225-232 is a review article wherein different semi-solid materials are described, such as polysaccharides, lipid based systems, cellulose derivatives, starch and amylose, chitosan, alginate and carbomers.

However, there are some draw backs with the existing formulations and there is a need for new improved ones.

Simethicone is an orally administered anti-foaming agent used to reduce bloating, discomfort or pain caused by excessive gas, mainly swallowed air, with small amounts of hydrogen and methane in the stomach or intestines.

There is a need for new products containing either cadotril or a combination of cadotril and simethicone to be able to provide new products on the market which aim at helping consumers suffering from a disorder or disease within the gastro intestinal tract.

### SUMMARY OF THE INVENTION

The invention relates to the development of new improved Cadotril compositions, such as Racecadotril alone or in combination with Simethicone. Such compositions have improved properties compared to present compositions including improved uptake, bioavailability and/or controlling release, such as sustained or delayed release. By governing and controlling the release it is possible to achieve an intended profile of the pharmaceutical agent, such as a faster or longer effect depending on the intended use. By the use of few and less toxic excipients it is as well possible to obtain a composition that is not harmful for the subject.

The invention is defined by the claims. Any subject-matter falling outside the scope of the claims is provided for information purposes only.

In a first aspect the invention relates to the semi-solid composition of claim comprising at least one cadotril, such as racecadotril, and at least one liquid-lipid and/or propylene glycol, whereby the invented semi-solid composition solves one or more of the above defined problems, such as influencing the effect on onset as well as duration.

It has surprisingly been found that such a composition creates a special crystalline structure during manufacturing, which will give rise to a high surface area, which is substantially free from water and which reduces the stability problem with the active ingredients. Formulating racecadotril in an oil base or other suitable water free liquids as mentioned below provides protection from hydrolysis in addition to improved absorption, rapid onset of action and increased bioavailability. Increased bioavailability permits the use of lower doses of racecadotril to achieve an efficacious therapeutic window, thus minimizing potential side effects associated with the use of racecadotril.

One example how to make the cadotril, such as racecadotril, may be by mixing racecadotril with one or more suitable liquid solvent excipients, heating the mixture and mixing to dissolve cadotril, such as racecadotril, and to obtain a homogenized liquid mixture. The mixture is allowed to cool down with continuous mixing. Due to simultaneous mixing and cooling, cadotril such as racecadotril crystallizes to fine crystals. The produced mixture shows semisolid consistency which is believed being attributed to the friction forces between the fine crystals as well as crystals interactions with the other components.

The relates to a semi-solid composition as claimed comprising at least one cadotril, such as racecadotril, simethicone and at least one liquid-lipid and/or propylene glycol excipient, which may be substantially free from surfactants, such a composition will for the first time enable the possibility to facilitate the administration of both ingredients in one composition.

In a second aspect the invention relates to a dose unit comprising the composition of the invention with either cadotril, such as racecadotril, or cadotril, such as racecadotril, together with simethicone. Such doses will give rise to the possibility to provide higher concentrations/doses of the cadotril, such as racecadotril, in the doses compared to what is possible today as well as due to the increased bioavailability decrease the concentration/dose. By providing a prolonged release profile it will secure that plasma levels will be kept within the effective concentration range for a longer period of time. Such prolonged release plasma profile makes it possible to reduce dosing frequency as well as having once or twice daily dosing instead of three times daily dosing product available on the market. In addition, the prolonged release may help to avoid too high plasma concentration, which could lead to increased risk for adverse or unwanted effects. There is no product available on the market today providing the mixture of cadotril, such as racecadotril, and simethicone and which solves the above identified problems. By the use of the specific crystalline structure of racecadotril it is possible to load a high dose and still have a dose unit that is possible to swallow. However, part of the active ingredient in the crystalline network, about 1,5 wt% or less, is in the soluble state and thus the soluble part will give rise to a fast release and thereby a fast effect with a low dose, when medium chain triglyceride (MCT) is used as the liquid excipient.

Finally, the invention relates to the semi-solid composition(s) defined above or the dose unit defined above for the treatment of a subject suffering from a disease or disorder in the gastro intestinal tract, wherein the semi-solid compositions could be co-administered with one or more dietary fiber products.

### BRIEF DESCRIPTION OF THE DRAWINGS

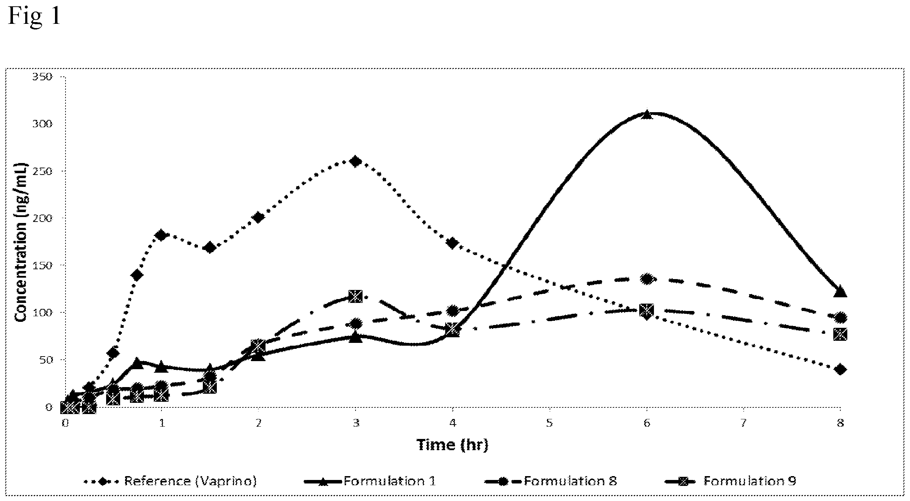

**Figure 1****:** Mean thiorphan plasma concentrations time curve following per oral administration of racecadotril semisolid formulations, alone or in combination with Simethicone at a dose of 20mg/kg in male beagle dogs study. A granulate powder from commercial capsule formulation, racecadotril (Vaprino100mg®) was used as a reference formulation for comparison. Each dog (n=12) was administered (P.O.) two capsules (equivalent to 200 mg racecadotril).

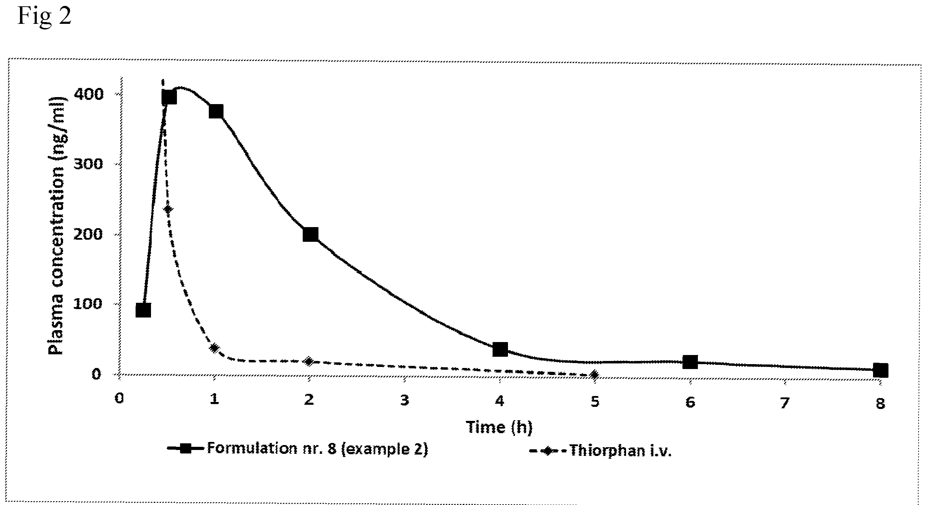

**Figure 2****:** Mean thiorphan plasma concentrations time curve in rat (n=3) following per oral administration of semisolid formulation of racecadotril in combination with Simethicone at a dose of 20mg/kg racecadotril and simethicone 25mg/kg [Formulation nr. 8 (example 2)]. A thiorphan i.v. (dose13.4 mg/kg) was used as a reference formulation for comparison.

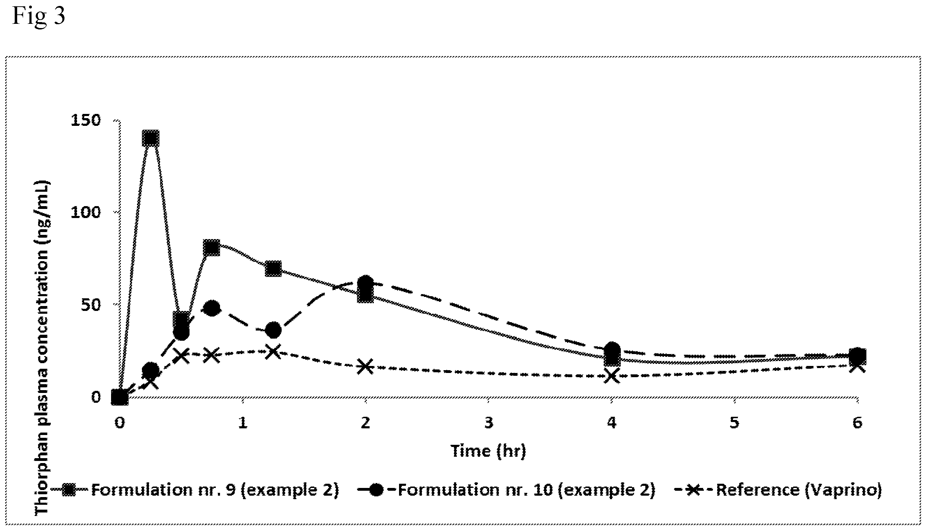

**Figure 3****:** Mean thiorphan plasma concentrations time curve in rat (n=5) following per oral administration of semisolid formulation of racecadotril in combination with Simethicone [Formulation nr. 9, 10 (example 2)] at a dose of 20mg/kg racecadotril and simethicone 25mg/kg. Racecadotril (Vaprino) at a dose of 20mg/kg was used as a reference formulation for comparison.

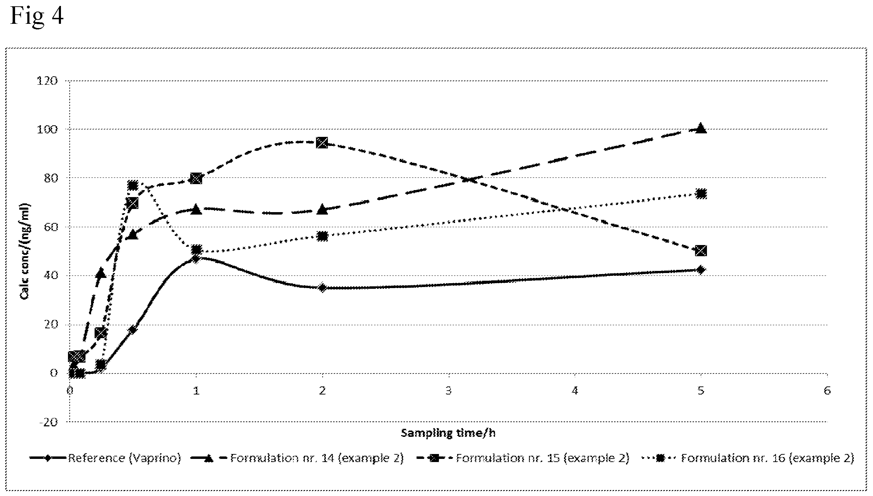

**Figure 4****:** Mean thiorphan plasma concentrations time curve in rat (n=5) following per oral administration of semisolid formulation of racecadotril in combination with Simethicone [Formulation nr. 14, 15 and 16 (example 2)] at a dose of 20mg/kg racecadotril and simethicone 25mg/kg. Racecadotril (Vaprino) at a dose of 20mg/kg was used as a reference formulation for comparison.

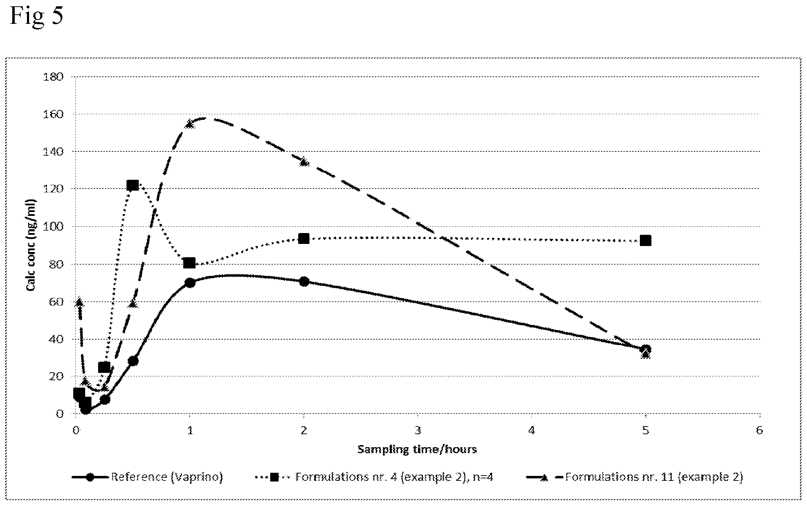

**Figure 5****:** Mean thiorphan plasma concentrations time curve in rat following per oral administration of racecadotril semisolid formulations, alone [Formulation nr. 4 (example 2), (n=4)] or in combination with Simethicone [Formulation nr. 11 (example 2), (n=5)] at a dose of 20mg/kg racecadotril and simethicone 25mg/kg. Racecadotril (Vaprino) at a dose of 20mg/kg was used as a reference formulation (n=5) for comparison.

### DETAILED DESCRIPTION OF EMBODIMENTS OF THE INVENTION

### Definitions

In the context of the present application and invention the following definitions apply:
The term Racecadotril, chemically known as benzyl N-[3-(acetylthio)-2 benzylpropanoyl] glycinate, is an anti-diarrheal drug which acts as a peripherally acting enkephalinase inhibitor. It has an anti-secretory effect and it is used to treat diarrhoea. It reduces the secretion of water and electrolytes into the intestine. Racecadotril is a prodrug and it is hydrolysed to its active metabolite, thiorphan following intravenous or oral administration.

The term semisolid is intended to mean the physical term for something that lies along the boundary between a solid and a liquid. While similar to a solid in some respects, in that semisolids can support their own weight and hold their shapes, a semisolid also shares some properties of liquids, such as conforming in shape to something applying pressure to it and the ability to flow under pressure. The words semisolid, quasisolid and semiliquid all mean exactly the same thing.

The term Medium Chain Triglyceride(s) (MCTs) is/are intended to mean triglycerides whose fatty acids have an aliphatic tail of 6-12 carbon atoms. The fatty acids found in MCTs are called medium-chain fatty acids (MCFAs). Like all triglycerides, MCTs are composed of a glycerol backbone and three fatty acids. In the case of MCTs, 2 or 3 of the fatty acid chains attached to glycerol are medium-chain in length. Examples includes, hexanoic acid (C6:0, common name caproic acid), octanoic acid (C8:0, common name caprylic acid), and decanoic acid (C10:0, common name capric acid) as well as dodecanoic acid (C12:0, common name lauric acid).

The term "substantially free from water or free from water" is intended to mean that the content of water present in the semi-solid composition is less than about 2 wt.% based on the total wt.% of the semi-solid composition, such as less than 1.5,1, 0.5, 0.4, 0.3, 0.2 or less than 0. 1 or totally free from water, i.e., 0 wt% based on the total wt.% of the semi-solid composition.

The term "substantially free from non-ionic surfactants or free from non-ionic surfactants" is intended to mean that the content of the non-ionic surfactant present in the semi-solid composition is less than about 2 wt.% based on the total wt.% of the semi-solid composition, such as less than 1.5,1, 0.5, 0.4, 0.3, 0.2 or less than 0.1 or totally free from surfactant, i.e., 0 wt.% based on the total wt.% of the semi-solid composition. The definition of a non-ionic surfactant is well-known for a person skilled in the art as being compounds that lower the surface tension (or interfacial tension) between two liquids or between a liquid and a solid. Non-ionic surfactants are amphiphilic molecules that have both a hydrophobic group non-polar "tail" and a hydrophilic group polar but uncharged "head". Prominent among these are the fatty alcohols, cetyl alcohol, stearyl alcohol, and cetostearyl alcohol (consisting predominantly of cetyl and stearyl alcohols), and oleyl alcohol.

The term "%w/w" is intended to mean the percentage of an mgredient(s)/ the total percentage by weight of the composition (100 %).

The term "bioavailability" is intended to mean, the rate and extent to which the active substance or active moiety is absorbed from a pharmaceutical form and becomes available at the site of action. Bioavailability of oral Racecadotril is assessed by monitoring concentrations of Racecadotril active metabolite (thiorphan) in the general circulation.

A "dosage", "dosage form", "dose unit" or "dose" as used herein means the amount of a pharmaceutical formulation comprising therapeutically active agent(s) administered at a time. "Dosage", "dosage form", "dose unit" or "dose" includes administration of one or more units of pharmaceutical formulation administered at the same time.

### The semi-solid composition

The invention relates to the semi-solid composition of claim 1 comprising at least one cadotril and at least one liquid-lipid and/or propylene glycol. The liquid-lipid excipient is at least one medium chain triglyceride or at least one oil containing medium chain triglycerides or a mixture thereof.

The semi-solid composition may be substantially free from non-ionic surfactants and/or substantially free from water as defined above. By not utilizing any non-ionic surfactant, there will be no problem with unpleasant taste, irritation or toxic effect which is common upon using surfactants.

The at least one medium chain triglyceride (MCT) is an ester of glycerol and a medium chain fatty acid, wherein the medium chain fatty acids are selected from the group consisting of caprylic acid (C8), caproic acid (C6), capric acid (C10), lauric acid (C12) or a mixture thereof. One example being that the medium chain triglyceride is an ester of glycerol and one or more medium chain fatty acids being caprylic or capric acid or a mixture thereof. Other examples are found in table 1.

The at least one medium chain triglyceride could be an ester of caprylic acid (C8), caproic acid (C6), capric acid (C10), or lauric acid (C12).

The medium chain fatty acid could be a natural oil containing medium chain fatty acids.

The at least one liquid-lipid and/or propylene glycol excipient is/are present in an amount of from 20 % w/w to 90 % w/w, from about 25 % w/w to about 90 % w/w, from about 40 % w/w to about 80 % w/w, from about 60 % w/w to about 70 % w/w, or from about 80 % w/w to about 90 % w/w of the total amount of the composition, such as 75% w/w, about 80% w/w or about 85% w/w.

The Medium chain fatty acid (MCFA) is one or more medium chain fatty acids selected from the group consisting of caprylic acid (C8), caproic acid (C6) acid, capric acid (C10), lauric acid (C12). The MCFA may be a mixture of caprylic and capric acid. The amount of the MCFA's in a MCT may be C6 < 2.0 %, C8 about 50-80 %, C10 about 20-50 %. One example is C12 <3.0 % and C14 <1.0% and the total amount of C8 and C10 up to 95% and the water content <0.2%.

Examples of MCTs presented on the market today are shown in table 1 below.

**Table 1. Examples of commercial MCT.**

| Product-Trade Mark | Supplier | Chemical description/INCI name | Listed in |
|---|---|---|---|
| Crodamol GTCC-LQ-(MV) | Croda | Caprylic/Capric Triglyceride | Triglycerides, Medium-Chain PhEur; |
| Old Trade Mark: Estasan GT8-60 3575 | | | Medium-Chain Triglycerides NF (Caprylic/Capric Triglycerides |
| MIGLYOL 812 | Sasol | Caprylic / Capric Triglyceride | Ph. Eur., USP-NF, JPE, DMF |
| LABRAFAC LIPOPHILE WL 1349 | Gattefossé | Triglycerides medium-chain EP/Medium -chain triglycerides NF/Medium chain fatty acid triglyceride JPE | DMF |
| | | | USP/NF |
| | | | EP |
| | | | JP/JPE |
| NEOBEE® M-5 | STEPAN | Captrin, Medium Chain Triglycerides, Caprylic/Capric Triglycerides or Glyceryl Tri(caprylate/caprate). | Complies with the specifications for Medium Chain Triglycerides of the National formulary as published by the U.S. Pharmacopoeia (USP 27/NF 22) and with EP and JPE. NEOBEE M-5 is Kosher and Halal Certified. NEOBEE M-5 has a Type IV Drug Master File (DMF) available. |
| CAPTEX® 355 | Abitec Corporation | Glycerol Tricaprylate/Caprate, Medium Chain Triglyceride (MCT); Caprylic/Capric Triglyceride; Octanoic/Decanoic Acid, Triglyceride | Meets current European Pharmacopoeia for Triglycerides, Medium-Chain, United States Pharmacopeia/National Formulary for Medium-Chain Triglyerides and Japanese Pharmaceutical Excipients monographs for Medium Chain (Fatty Acid) Triglycerides. |

The MCTs shown in table 1 above can be purchased from the following companies. Miglyol 812 from SASOL GmbH, CRODAMOL GTCC from Croda, or Neobees M-5 oil from Stepan and LABRAFAC LIPOPHILE WL 1349 from Gattefosse.

Examples of natural oils that could be used are all natural oils comprising MCTs, such as coconut or palm kernel oils.

In a first embodiment the invention relates to the semi-solid composition of claim 1 comprising at least one cadotril and at least one liquid-lipid and/or propylene glycol excipient. Examples and definition of the excipient being found above.

Example of cadotril includes racecadotril, dexecadotril and ecadotril or mixtures thereof. In the examples below racecadotril has been used. Cadotril(s) is/are present in an amount from 10 % w/w to 30% w/w, such as about 15% w/w, about 20% w/w or about 25% w/w. In one example the cadotril coexists in a dissolved and solid state present within the semi-solid composition.

The amount of the different ingredients present within the semi-solid composition may vary depending on which other components should be included.

The semi-solid composition of the invention may further comprise simethicone. Examples and definition of the excipient being found above. Example of cadotril includes racecadotril, dexecadotril and ecadotril or mixtures thereof. In the examples below racecadotril has been used. The amounts of cadotril being defined above.

Simethicone may be available from DOW CORNING(R) Q7-2243 LVA, SIMETHICONE USP, or DOW CORNING Antifoam M.

Simethicone is present in an amount of about 5 % w/w to about 75% w/w, about 5 % w/w to about 70% w/w, about 5 % w/w to about 60% w/w, 5% w/w to about 35% w/w, 10 % w/w to about 35% w/w, such as about 18,75% w/w, about 25% w/w or about 31,25% w/w.

The embodiments may further comprise one or more ingredient(s) selected from the list consisting of coloring agents, antioxidants, flavoring agents, sweeteners, thickeners, emulsifiers, excipients, preservatives and gelling agents.

Additionally, the composition may comprise one or more fibres, such as dietary fibre which consists of non-starch polysaccharides such as arabinoxylans, cellulose, and many other plant components such as resistant starch, resistant dextrins, inulin, lignin, waxes, chitins, pectins, beta-glucans, and oligosaccharides and other types of carbohydrate that the body can't digest and simply passes through the entire digestive tract. Generally, fibres come from plant foods: fruits, vegetables, grains, nuts, and legumes. Such fibres are classified as soluble fibres such as psyllium fibres, others are classified as insoluble fibres like those found in the seeds and skins of fruit as well as whole-wheat bread and brown rice.

The composition of the invention may further comprise an additional active ingredient. The additional active ingredient may be a digestive health active ingredient, for example, laxatives, antacids, proton pump inhibitors, anti-gas agents, antiemetics, H2 blockers, a second antidiarrheal agent, and the like. Examples of additional agents inlcude loperamide, α-galactosidase enzyme, calcium carbonate, aluminum hydroxide and magnesium hydroxide.

### A dose Unit

The invention also relates to a dose unit, wherein the dose unit comprises the semi-solid composition according to the invention. Cadotril(s), such as racecadotril, dexecadotril and ecadotril or mixtures thereof is/are present in an amount from about 5 mg to about 200 mg, such as about 5 mg or about 100 mg and simethicone is present in an amount from about 50 to about 1500 mg, such as about 50 to about 1000 mg, such as about 50 to about 500 mg, such as about 50 to about 100 mg, such as about 150 mg, or such as a about 125 mg. The dosage form may be a tablet or capsule.

### USE AND METHOD OF TREATMENT

Further the invention relates to the semi-solid composition as defined above or the dose unit defined above for the treatment of a subject suffering from a disease or disorder in the gastro intestinal tract, such as IBS, such as diarrhea and/or constipation and/or bloating, discomfort or pain caused by excessive gas.

The invention may be used in a method of treatment of a subject suffering from a disease or disorder in the gastro intestinal tract, such as IBS, such as diarrhea and/or constipation and/or bloating, discomfort or pain caused by excessive gas.

Following examples are intended to illustrate, but not to limit, the invention in any manner, shape, or form, either explicitly or implicitly.

### EXAMPLES 1: Preparation of bulk semi-solid formulation to be used in the examples below.

1. Racecadotril is weighed in a scintillation vial.
2. The MCT (obtained from Croda) is weighed and added in the same vial.
3. The mixture is heated in a water bath at 80°C (the vial is closed to avoid direct contact with water or water vapor) and mixed using vortex or homogenizer to dissolve racecadotril and achieve a clear homogenous solution.
4. Cooling down at room temperature with continuous mixing in order to obtain a semi-solid formulation.

Simethicone can be added either before heating and mixing or to be mixed with the semisolid at the end.

### Example 2: Formulations

| **Formulation nr.** | **Composition (% w/w)** |
|---|---|
| 1 | Racecadotril 15%, MCT 85% |
| 2 | Racecadotril 20%, MCT 80% |
| 3 | Racecadotril 25%, MCT 75% |

| **Formulation nr.** | **Composition (% w/w)** |
|---|---|
| 4 | Racecadotril 15%, propylene glycol (PG) 85% |
| 5 | Racecadotril 20%, propylene glycol (PG) 80% |
| 6 | Racecadotril 25%, propylene glycol (PG) 75% |
| 7 | Racecadotril 30%, propylene glycol (PG) 70% |

| **Formulation nr.** | **Composition (%)** |
|---|---|
| 8 | Racecadotril 15%, Simethicone 18.75%, MCT 66,25% |
| 9 | Racecadotril 20%, Simethicone 25% in MCT 55% |
| 10 | Racecadotril 25%, Simethicone 31.25% in MCT 43.75% |

| **Formulation nr.** | **Composition (%)** |
|---|---|
| 11 | Racecadotril 15%, Simethicone 18.75%, PG 66,25% |
| 12 | Racecadotril 20%, Simethicone 25%, PG 55% |
| 13 | Racecadotril 25%, Simethicone 31.25%, PG 43.75% |

| **Formulation nr.** | **Composition (%)** |
|---|---|
| 14 | Racecadotril 10%, Simethicone 20%, MCT 70% |
| 15 | Racecadotril 10%, Simethicone 40%, MCT 50% |
| 16 | Racecadotril 10%, Simethicone 60%, MCT 30% |

### EXAMPLE 3: Stability studies

Formulations nr. 1, 8 and 9 (example 2) were included in stability studies and have demonstrated stability up to 3 months at 25, 40 and 50°C with 98-100% nominal dose remaining.

### EXAMPLE 4: Oral administration crossover pharmacokinetic studies in male beagle dogs

Formulations nr. 1, 8 and 9 (example 2) were administered to dogs p.o. at doses expected to be pharmacologically effective. For estimation of the bioavailability, one group of dogs was given the reference product, a granulate powder from commercial capsule formulation, racecadotril (Vaprino® 100mg). Blood was drawn at specified time points and plasma concentration of thiorphan was assessed.

### Study Parameters:

**Animals:** male beagle dogs (N=12) of similar age (2 - 5.5 yrs) and body weight (9-12 kg) were selected to receive each formulation.

### Animal preparation:

Each dog was injected intramuscularly (IM) with pentagastrin solution ∼30 mins prior to dosing to maintain the stomach pH ∼1.2, which is similar to human.

Each dog was administered (P.O.) two capsules (equivalent to 200 mg racecadotril) followed by a dosing flush of 100 mL of sterile water.

The washout period between dosing each formulation was 5 days. Blood samples were collected at pre-determined time points (2, 5, 15, 30, 45 min, 1, 1.5, 2, 3, 4, 6 and 8 hours) and centrifuged at 4°C with 3000xg for 5 mins.

Plasma samples were transferred into appropriate storage vials and treated with the derivatizing reagent 2-bromo-3-methoxyacetophenone (BMP, 0.5 M in acetonitrile) for 10 mins prior to being immediately frozen on dry ice to stabilize the thiorphan. Plasma samples were then analyzed by LC-MS/MS.

Pharmacokinetic parameters (i.e., AUC, Cₘₐₓ, Tₘₐₓ, T_{1/2}, Kel, MRT) were calculated with WinNonlin® software using a non-compartmental model.

### Results:

Figure 1 shows the mean thiorphan plasma concentrations versus time curve following per oral administration of racecadotril formulations, alone or in combination with Simethicone at a dose of 20mg/kg in male beagle dogs study. A granulate powder from commercial capsule formulation, racecadotril (Vaprino100mg®) was used as a reference formulation for comparison.

The results of calculation for pharmacokinetic parameters are shown in tables (2, 3,4 and 5). Prolonged absorption profile was observed in terms of Cₘₐₓ and Tₘₐₓ. For formulations nr1, 8 and 9 (example 2) Cₘₐₓ was delayed for as long as 6 hours as compared to reference where Tₘₐₓ was approximately 3hours.

**Table 2. Individual and Average Pharmacokinetic Parameters for Thiorphan After Oral Administration of racecadotril reference formulation (Vaprino100mg®) in Male Beagle Dogs (200 mg Racecadotril/dog)**

| **Racecadotril reference formulation (Vaprino100mg ®)** | | | | | | | | | | | | | | |
|---|---|---|---|---|---|---|---|---|---|---|---|---|---|---|
| **Time (hr)** | **Dog #** | | | | | | | | | | | | | |
| | **146** | **147** | **148** | **149** | **150** | **151** | **152** | **153** | **154** | **155** | **156** | **157** | **Mean** | **SD** |
| **Animal Weight (kg)** | 11.9 | 11.7 | 9.4 | 8.4 | 8.4 | 9.2 | 8.6 | 8.6 | 9.6 | 8.4 | 10.0 | 10.0 | 9.5 | 1.2 |
| **Dose (mg/kg)** | 16.8 | 17.1 | 21.3 | 23.8 | 23.8 | 21.7 | 23.3 | 23.3 | 20.8 | 23.8 | 20.0 | 20.0 | 21.3 | 2.5 |
| **Cₘₐₓ (ng/mL)** | 366 | 432 | 328 | 478 | 152 | 273 | 303 | 499 | 168 | 10.9 | 334 | 356 | 308 | 142 |
| **tₘₐₓ (hr)** | 2.0 | 1.0 | 3.0 | 3.0 | 3.0 | 1.0 | 1.5 | 3.0 | 1.0 | 4.0 | 3.0 | 2.0 | 2.3 | 1.0 |
| **t_{1/2} (hr)** | ND² | 1.21 | 1.07 | 3.41 | 2.26 | ND² | ND² | 1.93 | 1.55 | ND³ | 1.28 | ND² | 1.81 | 0.818 |
| **MRTₗₐₛₜ (hr)** | 2.84 | 2.69 | 3.21 | 3.31 | 3.48 | 3.28 | 3.76 | 4.18 | 3.22 | 2.03 | 3.92 | 3.30 | 3.27 | 0.571 |
| **AUCₗₐₛₜ (hr·ng/mL)** | 991 | 1431 | 1354 | 1241 | 513 | 1020 | 1340 | 1836 | 661 | 21.4 | 1302 | 1126 | 1070 | 481 |
| **AUC_{∞} (hr·ng/mL)** | ND² | 1462 | 1386 | 1520 | 595 | ND² | ND² | 2068 | 702 | ND³ | 1368 | ND² | 1300 | 505 |

| **Dose-normalized Values¹** | | | | | | | | | | | | | | |
|---|---|---|---|---|---|---|---|---|---|---|---|---|---|---|
| **AUCₗₐₛₜ (hr·kg·ng/mL/mg)** | 59.0 | 83.7 | 63.5 | 52.2 | 21.5 | 47.0 | 57.5 | 78.8 | 31.8 | 0.899 | 65.1 | 56.3 | 51.4 | 23.5 |
| **AUC_{∞} (hr·kg·ng/mL/mg)** | ND² | 85.5 | 65.1 | 63.9 | 25.0 | ND² | ND² | 89 | 33.76 | ND³ | 68.4 | ND² | 61.5 | 24.1 |

| | | | | | | | | | | | | | | |
|---|---|---|---|---|---|---|---|---|---|---|---|---|---|---|
| Cₘₐₓ: Maximum plasma concentration; tₘₐₓ: Time of maximum plasma concentration; t_{1/2}: half-life, data points used for half-life determination are in bold in the respective plasma concentration table; MRT: mean residence time; AUCₗₐₛₜ: Area Under the Curve, calculated to the last observable time point; AUC_{∞}: Area Under the Curve, extrapolated to infinity; ND: Not Determined; ¹Dose-normalized by dividing the parameter by the dose of racecadotril in mg/kg; ²not determined because the terminal elimination phase had an R² of less than 0.85; ³not determined because of a lack of quantifiable data points trailing the Cₘₐₓ. | | | | | | | | | | | | | | |

**Table 3. Individual and Average Pharmacokinetic Parameters for Thiorphan After Oral Administration of Formulation nr. 1 (example 2) in Male Beagle Dogs (200 mg Racecadotril/dog)**

| **Formulation nr. 1 (example 2)** | | | | | | | | | | | | | | |
|---|---|---|---|---|---|---|---|---|---|---|---|---|---|---|
| **Time (hr)** | **Dog #** | | | | | | | | | | | | | |
| | **146** | **147** | **148** | **149** | **150** | **151** | **152** | **153** | **154** | **155** | **156** | **157** | **Mean** | **SD** |
| **Animal Weight (kg)** | 10.4 | 10.9 | 9.1 | 8.5 | 7.7 | 9.8 | 8.7 | 8.6 | 9.5 | 8.6 | 10.2 | 10.2 | 9.4 | 1.0 |
| **Dose (mg/kg)** | 19.2 | 18.3 | 22.0 | 23.5 | 26.0 | 20.4 | 23.0 | 23.3 | 21.1 | 23.3 | 19.6 | 19.6 | 21.6 | 2.3 |
| **Cₘₐₓ (ng/mL)** | 245 | 232 | 69.1 | 243 | 188 | 141 | 206 | 1340* | 120 | 810 | 141 | 10.1* | 240 | 209 |
| **tₘₐₓ (hr)** | 0.75 | 3.0 | 1.0 | 6.0 | 4.0 | 8.0 | 6.0 | 6.0* | 8.0 | 6.0 | 6.0 | 0.25* | 4.9 | 2.3 |
| **t_{1/2} (hr)** | ND² | ND² | ND² | ND² | ND² | ND² | ND² | ND² | ND² | ND² | ND² | ND³ | ND | ND |
| **MRTₗₐₛₜ (hr)** | 3.93 | 4.90 | 4.31 | 5.60 | 5.19 | 4.82 | 5.54 | 5.85* | 4.88 | 5.94 | 5.03 | ND³ | 5.01 | 0.601 |
| **AUCₗₐₛₜ (hr·ng/mL)** | 653 | 1135 | 359 | 584 | 759 | 666 | 788 | 3040* | 661 | 2002 | 770 | ND³ | 838 | 453 |
| **AUC_{∞} (hr·ng/mL)** | ND² | ND² | ND² | ND² | ND² | ND² | ND² | ND² | ND² | ND² | ND² | ND³ | ND | ND |

| **Dose-normalized Values¹** | | | | | | | | | | | | | | |
|---|---|---|---|---|---|---|---|---|---|---|---|---|---|---|
| **AUCₗₐₛₜ (hr·kg·ng/mL/mg)** | 34.0 | 62.0 | 16.3 | 24.9 | 29.2 | 32.7 | 34.3 | 130* | 31.3 | 85.9 | 39.3 | ND³ | 39.0 | 20.2 |
| **AUC_{∞} (hr·kg·ng/mL/mg)** | ND² | ND² | ND² | ND² | ND² | ND² | ND² | ND² | ND² | ND² | ND² | ND³ | ND | ND |

| | | | | | | | | | | | | | | |
|---|---|---|---|---|---|---|---|---|---|---|---|---|---|---|
| Cₘₐₓ: Maximum plasma concentration; tₘₐₓ: Time of maximum plasma concentration; t_{1/2}: half-life, data points used for half-life determination are in bold in the respective plasma concentration table; MRT: mean residence time; AUCₗₐₛₜ: Area Under the Curve, calculated to the last observable time point; ²not determined because the terminal elimination phase was not observed; ⁴not determined due to a lack of quantifiable data points; *value removed as an outlier, see Appendix E. | | | | | | | | | | | | | | |

**Table 4. Individual and Average Pharmacokinetic Parameters for Thiorphan After Oral Administration of Formulation nr. 8 (example 2) in Male Beagle Dogs (200 mg Racecadotril/dog)**

| **Formulation nr. 8 (example 2)** | | | | | | | | | | | | | | |
|---|---|---|---|---|---|---|---|---|---|---|---|---|---|---|
| **Time (hr)** | **Dog #** | | | | | | | | | | | | | |
| | **146** | **147** | **148** | **149** | **150** | **151** | **152** | **153** | **154** | **155** | **156** | **157** | **Mean** | **SD** |
| **Animal Weight (kg)** | 11.1 | 10.7 | 9.5 | 8.4 | 8.0 | 9.6 | 8.3 | 9.0 | 9.5 | 8.2 | 9.7 | 9.7 | 9.3 | 1.0 |
| **Dose (mg/kg)** | 18.0 | 18.7 | 21.0 | 23.8 | 25.0 | 20.8 | 24.1 | 22.2 | 21.1 | 24.4 | 20.6 | 20.6 | 21.7 | 2.2 |
| **Cₘₐₓ (ng/mL)** | 38.8 | 180 | 148 | 134 | 203 | 152 | 213 | 308 | 168 | 139 | 110 | ND³ | 163 | 67.4 |
| **tₘₐₓ (hr)** | 8.0 | 6.0 | 8.0 | 4.0 | 4.0 | 2.0 | 3.0 | 6.0 | 4.0 | 8.0 | 4.0 | ND³ | 5.2 | 2.1 |
| **t_{1/2} (hr)** | ND² | ND² | ND² | ND² | ND² | ND² | ND² | ND² | ND² | ND² | ND² | ND³ | ND | ND |
| **MRTₗₐₛₜ (hr)** | 4.73 | 4.98 | 5.18 | 4.85 | 5.00 | 4.34 | 4.75 | 5.52 | 4.79 | 4.71 | 4.58 | ND³ | 4.86 | 0.312 |
| **AUCₗₐₛₜ (hr·ng/mL)** | 181 | 932 | 647 | 619 | 651 | 424 | 861 | 1086 | 845 | 752 | 617 | ND³ | 692 | 248 |
| **AUC∞ (hr·ng/mL)** | ND² | ND² | ND² | ND² | ND² | ND² | ND² | ND² | ND² | ND² | ND² | ND³ | ND | ND |

| **Dose-normalized Values¹** | | | | | | | | | | | | | | |
|---|---|---|---|---|---|---|---|---|---|---|---|---|---|---|
| **AUCₗₐₛₜ(hr·kg·ng/mL/mg)** | 10.1 | 49.9 | 30.8 | 26.0 | 26.0 | 20.4 | 35.7 | 48.9 | 40.1 | 30.8 | 29.9 | ND³ | 31.7 | 11.7 |
| **AUC_{∞} (hr·kg·ng/mL/mg)** | ND² | ND² | ND² | ND² | ND² | ND² | ND² | ND² | ND² | ND² | ND² | ND³ | ND | ND |

| | | | | | | | | | | | | | | |
|---|---|---|---|---|---|---|---|---|---|---|---|---|---|---|
| Cₘₐₓ: Maximum plasma concentration; tₘₐₓ: Time of maximum plasma concentration; t_{1/2}: half-life, data points used for half-life determination are in bold in the respective plasma concentration table; MRT: mean residence time; AUCₗₐₛₜ: Area Under the Curve, calculated to the last observable time point; ²not determined because the terminal elimination phase was not observed; ⁴not determined due to a lack of quantifiable data points. | | | | | | | | | | | | | | |

**Table 5. Individual and Average Pharmacokinetic Parameters for Thiorphan After Oral Administration of Formulation nr. 9 (example 2) in Male Beagle Dogs (200 mg Racecadotril/dog)**

| **Formulation nr. 9 (example 2)** | | | | | | | | | | | | | | |
|---|---|---|---|---|---|---|---|---|---|---|---|---|---|---|
| **Time (hr)** | **Dog #** | | | | | | | | | | | | | |
| | **146** | **147** | **148** | **149** | **150** | **151** | **152** | **153** | **154** | **155** | **156** | **157** | **Mean** | **SD** |
| **Animal Weight (kg)** | 11.4 | 11.8 | 9.8 | 9.0 | 8.8 | 10.0 | 9.3 | 9.3 | 10.4 | 9.0 | 8.8 | 10.0 | 9.8 | 1.0 |
| **Dose (mg/kg)** | 17.5 | 16.9 | 20.4 | 22.2 | 22.7 | 20.0 | 21.5 | 21.5 | 19.2 | 22.2 | 22.7 | 20.0 | 20.6 | 1.9 |
| **Cₘₐₓ (ng/mL)** | 150 | 139 | 75.7 | ND⁴ | 156 | 396 | 80.8 | 232 | 101 | 251 | 260 | 153 | 181 | 95.7 |
| **tₘₐₓ (hr)** | 8.0 | 6.0 | 1.5 | ND⁴ | 6.0 | 3.0 | 8.0 | 6.0 | 6.0 | 2.0 | 3.0 | 3.0 | 4.8 | 2.3 |
| **t_{1/2} (hr)** | ND³ | ND³ | ND² | ND⁴ | ND³ | ND² | ND³ | ND³ | ND³ | ND² | 1.35 | 3.22 | 2.29 | ND |
| **MRTₗₐₛₜ (hr)** | 6.51 | 5.30 | 3.79 | ND⁴ | 5.57 | 4.12 | 5.32 | 5.52 | 5.54 | 4.61 | 3.57 | 4.45 | 4.94 | 0.898 |
| **AUCₗₐₛₜ (hr·ng/mL)** | 255 | 579 | 257 | ND⁴ | 548 | 933 | 277 | 805 | 445 | 978 | 878 | 520 | 589 | 273 |
| **AUC_{∞} (hr·ng/mL)** | ND³ | ND³ | ND² | ND⁴ | ND³ | ND² | ND³ | ND³ | ND³ | ND² | 922 | ND⁵ | ND | ND |

| **Dose-normalized Values¹** | | | | | | | | | | | | | | |
|---|---|---|---|---|---|---|---|---|---|---|---|---|---|---|
| **AUCₗₐₛₜ(hr·kg·ng/mL/mg)** | 14.6 | 34.3 | 12.6 | ND⁴ | 24.1 | 46.6 | 12.9 | 37.4 | 23.2 | 44.1 | 38.7 | 26.0 | 28.6 | 12.4 |
| **AUC_{∞} (hr·kg·ng/mL/mg)** | ND³ | ND³ | ND² | ND⁴ | ND³ | ND² | ND³ | ND³ | ND³ | ND² | 40.6 | ND⁵ | ND | ND |

| | | | | | | | | | | | | | | |
|---|---|---|---|---|---|---|---|---|---|---|---|---|---|---|
| Cₘₐₓ: Maximum plasma concentration; tₘₐₓ: Time of maximum plasma concentration; t_{1/2}: half-life, data points used for half-life determination are in bold in the respective plasma concentration table; MRT: mean residence time; AUCₗₐₛₜ: Area Under the Curve, calculated to the last observable time point; AUC_{∞}: Area Under the Curve, extrapolated to infinity; ND: Not Determined; ¹Dose-normalized by dividing the parameter by the dose of racecadotril in mg/kg; ²not determined because the terminal elimination phase had an R² of less than 0.85; ³not determined because the terminal elimination phase was not observed; ⁴not determined due to a lack of quantifiable data points; ⁵not determined because the AUC_{∞} was a greater than 25% extrapolation above the AUCₗₐₛₜ | | | | | | | | | | | | | | |

### EXAMPLE 5: oral administration pharmacokinetic studies in rats

Formulations were administered to rats at doses expected to be pharmacologically effective for estimation of the bioavailability. Blood was drawn at specified time points and plasma concentration of thiorphan was assessed.

### Study Parameters:

**Animals:** Sprague Dawley rats, approximate weight 250 g.

**Animal preparation:** Deprived of food (but not of water) during 18 h preceding dosing.

**Mode of administration:** thiorphan (i.v.) and racecadotril (p.o.) by oral gavage.

**Blood sampling volume:** 300 µl/per sample.

Blood was drawn at specified time points. Plasma was immediately frozen and kept at -80°C for assessment of thiorphan concentration by HPLC.

### EXAMPLE 5A:

Formulation nr. 8 (example 2) was administered to rats p.o. at dose of 20mg/kg. One group of rats was given thiorphan i.v. at a dose of 13.4 mg/kg as a reference.

### Results:

Figure 2 and Table 6 show plasma concentration results, increased bioavailability was observed in terms of AUC and Cₘₐₓfor formulation nr 8 (example 2).

**Table 6. Individual and Average Pharmacokinetic Parameters for Thiorphan. Formulation nr. 8 (example 2) was administered to rats p.o. at dose of 20mg/kg. One group of rats was given thiorphan i.v. at a dose of 13.4 mg/kg as a reference.**

| **Treatment** | **Subject** | T_**half** | **Tmax** | **Cmax** | **AUCt** | **AUCinf** | **Rel BA (%)** |
|---|---|---|---|---|---|---|---|
| | | (hr) | (hr) | (ng/mL) | (ng/mL*hr) | (ng/mL*hr) | % |
| IV_A **Thiorphan i.v.** | 1 | 1.81 | 0.033 | 21914 | 857 | 871 | |
| | 2 | 1.31 | 0.033 | 15924 | 2392 | 2403 | |
| | 3 | 0.57 | 0.033 | 17649 | 1843 | 1857 | |
| | | | | | Avg | ***1710*** | 100 |

| **Formulation nr. 8 (example 2)** | | | | | | | |
|---|---|---|---|---|---|---|---|
| (15% racecadotril, 18.8% Simethicone, MCT ad PO_S q.s.) | 1 | 2.14 | 1.000 | 195 | 616 | 664 | |
| | 2 | 0.73 | 0.500 | 436 | 650 | 653 | |
| | 3 | 1.85 | 1.000 | 615 | 1118 | 1147 | |
| | | | | | Avg | ***822*** | 32 |

| | | | | | | | |
|---|---|---|---|---|---|---|---|
| *non-compartment analysis | | | | | | | |

### EXAMPLE 5B:

Formulations nr. 9 and 10 (example 2) were administered to rats p.o. at dose of 20mg/kg. One group of rats was given reference product, a granulate powder from commercial capsule formulation, racecadotril (Vaprino100mg®).

### Results:

Figure 3 shows plasma concentration results, increased bioavailability was observed in terms of AUC and Cₘₐₓfor formulations nr 9 and 10 (example 2) as compared to reference.

### EXAMPLE 5C:

Formulations with different ratios of simethicone were tested. Formulations nr. 14, 15 and 16, (example 2) were administered to rats p.o. at dose of 20mg/kg. One group of rats was given reference product, a granulate powder from commercial capsule formulation, racecadotril (Vaprino100mg ®).

### Results:

Figure 4 shows plasma concentration results. Both immediate and prolonged release profiles were observed as well as an increased bioavailability in terms of AUC and Cₘₐₓ was observed for formulations nr 14, 15 and 16 (example 2) as compared to reference.

### EXAMPLE 5D:

Propylene glycol based formulations of racecadotril, alone or in combination with simethicone were tested. Formulations nr. 4, and 11, (example 2) were administered to rats p.o. at dose of 20mg/kg. One group of rats was given reference product, a granulate powder from commercial capsule formulation, racecadotril (Vaprino100mg®).

### Results:

Figure 5 shows plasma concentration results. Immediate release profile as well as increased bioavailability in terms of AUC and Cₘₐₓ were observed for both formulations nr. 4, and 11, (example 2). In addition, a prolonged absorption profile was observed with formulation nr 4 (example 2) as compared to reference.

## Claims

1. A semi-solid composition comprising
a. at least one cadotril in an amount from 10 wt.% to 30 wt.%, and
b. at least one liquid-lipid and/or propylene glycol excipient, wherein the liquid-lipid excipient is at least one medium chain triglyceride or at least an oil containing medium chain triglycerides or mixtures thereof; and wherein component b is/are present in an amount of from 20 wt.% to 90 wt.%.

2. The semi-solid composition according to claim 1, wherein the content of water present in the semi-solid composition is less than 2 wt. %, and/or the content of non-ionic surfactant present in the semi-solid composition is less than 2 wt.% .

3. The semi-solid composition according to any of preceding claims, wherein the medium chain triglyceride is at least an ester of glycerol and at least one or more medium chain fatty acids selected from the group consisting of caprylic acid (C8), caproic acid (C6), capric acid (C10), lauric acid (C12) or a mixture thereof.

4. The semi-solid composition according to claim 3, wherein the medium chain triglyceride is an ester of glycerol and one or more medium chain fatty acids being caprylic or capric acid or a mixture thereof.

5. The semi-solid composition according to any of preceding claims, wherein the cadotril coexists in a dissolved and solid state.

6. The semi-solid composition according to any of preceding claims, wherein the at least one cadotril is selected from the group consisting of racecadotril, dexecadotril and ecadotril or mixtures thereof.

7. The semi-solid composition according to claim 6, wherein the cadotril is racecadotril.

8. The semi-solid composition according to any of preceding claims, wherein the composition further comprises simethicone.

9. The semi-solid composition according to any of preceding claims, further comprising simethicone in an amount from 5 % w/w to 75% w/w.

10. The semi-solid composition according to any of preceding claims, wherein the composition further comprises one or more ingredient(s) selected from the list consisting of colorings, flavors, sweeteners, thickeners, emulsifiers, antioxidants, preservatives, gelling agents and disintegrants.

11. The semi-solid composition according to any of preceding claims, wherein said composition comprises at least one dietary fibre.

12. A dose unit comprising the composition according to any of preceding claims.

13. The dose unit according to claim 12, wherein the cadotril(s) is/are present in an amount from 5 mg to 200 mg.

14. The dose unit according to claims 12 - 13, wherein simethicone is present in an amount from 50 mg to 1500 mg.

15. The semi-solid composition according to any of claims 1-11, or the dose unit according to any of claims 12 - 14, for use in the treatment of a subject suffering from a disease or disorder in the gastro intestinal tract.

## Patentansprüche

1. Halbfeste Zusammensetzung, umfassend
a. mindestens ein Cadotril in einer Menge von 10 Gew.-% bis 30 Gew.-% und
b. mindestens einen flüssigen Lipidexzipienten und/oder einen Propylenglykolexzipienten, wobei es sich bei dem flüssigen Lipidexzipienten um mindestens ein Triglycerid mit mittlerer Kettenlänge oder mindestens ein Triglyzeride mit mittlerer Kettenlänge oder Mischungen davon enthaltendes Öl handelt und wobei Komponente b in einer Menge von 20 Gew.-% bis 90 Gew.-% vorliegt.

2. Halbfeste Zusammensetzung nach Anspruch 1, wobei der Gehalt an in der halbfesten Zusammensetzung vorhandenem Wasser weniger als 2 Gew.-% beträgt und/oder der Gehalt an in der halbfesten Zusammensetzung vorhandenem nichtionischem Tensid weniger als 2 Gew.-% beträgt.

3. Halbfeste Zusammensetzung nach einem der vorhergehenden Ansprüche, wobei es sich bei dem Triglycerid mit mittlerer Kettenlänge um mindestens einen Ester von Glycerin und mindestens einer oder mehreren Fettsäuren mit mittlerer Kettenlänge ausgewählt aus der aus Caprylsäure (C8), Capronsäure (C6), Caprinsäure (C10), Laurinsäure (C12) oder einer Mischung davon bestehenden Gruppe handelt.

4. Halbfeste Zusammensetzung nach Anspruch 3, wobei es sich bei dem Triglycerid mit mittlerer Kettenlänge um einen Ester von Glycerin und einer oder mehreren Fettsäuren mit mittlerer Kettenlänge, bei denen es sich um Caprylsäure oder Caprinsäure oder eine Mischung davon handelt, handelt.

5. Halbfeste Zusammensetzung nach einem der vorhergehenden Ansprüche, wobei das Cadotril in gelöstem und festem Zustand coexistiert.

6. Halbfeste Zusammensetzung nach einem der vorhergehenden Ansprüche, wobei das mindestens eine Cadotril aus der aus Racecadotril, Dexecadotril und Ecadotril oder Mischungen davon bestehenden Gruppe ausgewählt ist.

7. Halbfeste Zusammensetzung nach Anspruch 6, wobei es sich bei dem Cadotril um Racecadotril handelt.

8. Halbfeste Zusammensetzung nach einem der vorhergehenden Ansprüche, wobei die Zusammensetzung weiterhin Simethicon umfasst.

9. Halbfeste Zusammensetzung nach einem der vorhergehenden Ansprüche, weiterhin umfassend Simethicon in einer Menge von 5 Gew.-% bis 75 Gew.-%.

10. Halbfeste Zusammensetzung nach einem der vorhergehenden Ansprüche, wobei die Zusammensetzung weiterhin einen oder mehrere Bestandteile ausgewählt aus der aus Farbstoffen, Geschmacksstoffen, Süßstoffen, Verdickungsmitteln, Emulgatoren, Antioxidationsmitteln, Konservierungsstoffen, Geliermitteln und Sprengmitteln bestehenden Liste umfasst.

11. Halbfeste Zusammensetzung nach einem der vorhergehenden Ansprüche, wobei die Zusammensetzung mindestens einen Nahrungsmittelfaserstoff umfasst.

12. Dosierungseinheit, umfassend die Zusammensetzung nach einem der vorhergehenden Ansprüche.

13. Dosierungseinheit nach Anspruch 12, wobei das Cadotril/die Cadotrile in einer Menge von 5 mg bis 200 mg vorliegt/vorliegen.

14. Dosierungseinheit nach Anspruch 12 oder 13, wobei das Simethicon in einer Menge von 50 mg bis 1500 mg vorliegt.

15. Halbfeste Zusammensetzung nach einem der Ansprüche 1-11 oder Dosierungseinheit nach einem der Ansprüche 12-14 zur Verwendung bei der Behandlung eines an einer Krankheit bzw. Störung im Magen-Darm-Trakt leidenden Subjekts.

## Revendications

1. Composition semi-solide, comprenant
a. au moins un cadotril, selon une quantité allant de 10% en poids à 30% en poids, et
b. au moins un excipient de type lipide liquide et/ou propylène glycol, où l'excipient de type lipide liquide est constitué d'au moins un triglycéride à chaîne moyenne ou d'au moins une huile contenant des triglycérides à chaîne moyenne, ou des mélanges de ceux-ci ; et où le ou les composants b sont présents selon une quantité allant de 20% en poids à 90% en poids.

2. Composition semi-solide selon la revendication 1, dans laquelle la teneur en eau présente dans la composition semi-solide est inférieure à 2% en poids, et/ou la teneur en agent tensioactif non ionique présent dans la composition semi-solide est inférieure à 2% en poids.

3. Composition semi-solide selon l'une quelconque des revendications précédentes, dans laquelle le triglycéride à chaîne moyenne est constitué d'au moins un ester de glycérol et d'au moins un ou plusieurs acides gras à chaîne moyenne choisis dans le groupe constitué par l'acide caprylique (C8), l'acide caproïque (C6), l'acide caprique (C10), l'acide laurique (C12) ou un mélange de ceux-ci.

4. Composition semi-solide selon la revendication 3, dans laquelle le triglycéride à chaîne moyenne est constitué d'un ester de glycérol et d'un ou plusieurs acides gras à chaîne moyenne qui sont l'acide caprylique ou caprique, ou un mélange de ceux-ci.

5. Composition semi-solide selon l'une quelconque des revendications précédentes, dans laquelle le cadotril coexiste sous forme dissoute et solide.

6. Composition semi-solide selon l'une quelconque des revendications précédentes, dans laquelle le au moins un cadotril est choisi dans le groupe constitué par le racécadotril, le dexécadotril et l'écadotril, ou des mélanges de ceux-ci.

7. Composition semi-solide selon la revendication 6, dans laquelle le cadotril est le racécadotril.

8. Composition semi-solide selon l'une quelconque des revendications précédentes, la composition comprenant en outre de la siméthicone.

9. Composition semi-solide selon l'une quelconque des revendications précédentes, comprenant en outre de la siméthicone selon une quantité allant de 5% p/p à 75% p/p.

10. Composition semi-solide selon l'une quelconque des revendications précédentes, la composition comprenant en outre un ou plusieurs ingrédients choisis dans la liste constituée par les colorants, les arômes, les édulcorants, les épaississants, les émulsifiants, les antioxydants, les conservateurs, les agents gélifiants et les délitants.

11. Composition semi-solide selon l'une quelconque des revendications précédentes, ladite composition comprenant au moins une fibre alimentaire.

12. Dose unitaire, comprenant la composition selon l'une quelconque des revendications précédentes.

13. Dose unitaire selon la revendication 12, dans laquelle le ou les cadotrils sont présents selon une quantité allant de 5 mg à 200 mg.

14. Dose unitaire selon les revendications 12-13, dans laquelle la siméthicone est présente selon une quantité allant de 50 mg à 1500 mg.

15. Composition semi-solide selon l'une quelconque des revendications 1-11, ou dose unitaire selon l'une quelconque des revendications 12-14, pour une utilisation dans le traitement d'un sujet souffrant d'une maladie ou d'un trouble du tractus gastro-intestinal.
